# EUROPEAN PATENT SPECIFICATION

(11) **EP 4 203 888 B1**
(45) Date of publication and mention of the grant of the patent: **28.01.2026**
(21) Application number: 21862473.2
(22) Date of filing: 23.08.2021
(51) Int. Cl.: A61J 1/14, A61J 1/20, A61M 5/162, A61M 39/00, A61M 39/10, A61M 39/26

(54) **MEMBRANE FOR CLOSED SYSTEM TRANSFER DEVICE**
MEMBRAN FÜR EINE ÜBERTRAGUNGSVORRICHTUNG MIT GESCHLOSSENEM SYSTEM
MEMBRANE POUR DISPOSITIF DE TRANSFERT DE SYSTÈME FERMÉ

(30) Priority: 25.08.2020 US 202063070020 P
(43) Date of publication of application: 05.07.2023
(73) Proprietor: Becton, Dickinson and Company, Franklin Lakes, NJ 07417 (US)
(72) Inventor: PATEL, Romesh, Bridgewater, New Jersey 08807 (US)
(74) Representative: dompatent
(86) International application number: PCT/US2021/047143
(87) International publication number: WO 2022/046629

(56) References cited:
- EP-A1- 3 669 930
- US-A- 4 755 173
- US-A1- 2008 053 565
- US-A1- 2012 179 128
- US-A1- 2012 203 193
- US-A1- 2015 123 398
- US-B2- 9 089 475

## Description

### CROSS-REFERENCE TO RELATED APPLICATION

The present application claims priority to United States Provisional application Serial No. 63/070,020, entitled "Membrane for Closed System Transfer Device", filed August 25, 2020.

### BACKGROUND OF THE INVENTION

### Field of the Disclosure

The present disclosure relates generally to a membrane for a closed system transfer device.

### Description of the Related Art

Health care providers reconstituting, transporting, and administering hazardous drugs, such as cancer treatments, can put health care providers at risk of exposure to these medications and present a hazard in the health care environment. Unintentional chemotherapy exposure can affect the nervous system, impair the reproductive system, and bring an increased risk of developing blood cancers in the future. Some drugs must be dissolved or diluted before they are administered, which involves transferring a solvent from one container to a sealed vial containing the drug in powder or liquid form, by means of a needle. Drugs may be inadvertently released into the atmosphere in gas form or by way of aerosolization, during the withdrawal of the needle from the vial and while the needle is inside the vial if any pressure differential between the interior of the vial and the surrounding atmosphere exists. In order to reduce the risk of health care providers being exposed to toxic drugs, the transfer of these drugs is accomplished utilizing a closed system transfer device or system.

Closed system transfer devices or systems may utilize membranes to ensure the safe transfer of fluid between components. For example, a syringe adapter may include a membrane that contacts a membrane of a mating component, such as a patient connector, IV bag spike, or vial adapter.
EP 3 669 930 A1 discloses a pierceable septum for use in drug reservoirs and infusion sets. US 4,755,173 A discloses an injection set for delivering a fluid to a subcutaneous injection location in a patient.
US 2008/053565 discloses vial assembly which includes a storage vial, a stopper member and a securing ring.
US 2012/0203193 A1 discloses a fluid transfer system for transferring a substance from one vessel to another vessel while avoiding leakage of liquid and gas contaminations.

### SUMMARY OF THE INVENTION

According to the invention, a membrane for a closed system transfer device includes a first portion having a first material, and a second portion having a second material, with the first material having different material properties than the second material. The first portion is configured to prevent leakage through the first portion when the first portion is punctured by a cannula for a first predetermined amount of time, and the second portion is configured to prevent leakage through the second portion when the second portion is punctured by the cannula for a second predetermined amount of time. The first predetermined amount of time is larger than the second predetermined amount of time.

The first material is a thermoplastic elastomer. The second material is polyisoprene. The first predetermined amount of time may be one hour or greater. The second predetermined amount of time may be 10 seconds or shorter. The first portion and the second portion may be formed separately or integrally. The first portion includes a body having a first side and a second side positioned opposite the first side, with the first portion having a flange extending from the body. The second portion includes a body having a first side and a second side positioned opposite the first side, with the second portion having a flange extending from the body. The flange of the first portion abuts the flange of the second portion.

In a further aspect or embodiment, a patient connector includes a body having a first end and a second end, with the body defining a passageway, a line connection positioned at the second end of the body, and a membrane according to any of the aspects or embodiments discussed above, with the membrane positioned at the first end of the body.

The membrane may be received by an opening defined by the body. The opening may be wider than the passageway, with the body of the first portion extending from the first end of the body, the flange of the first portion positioned within the opening, the body of the second portion positioned within the passageway, and the flange of the second portion positioned within the opening. The body of the patient connector may include a securing extension at the first end of the body, with the securing extension extending radially inward and configured to secure the membrane to the body.

In another aspect or embodiment, a system for the closed transfer of fluid includes a patient connector according to any of the aspects or embodiments discussed above, and a syringe adapter having a housing with a syringe adapter membrane received within the housing and a cannula. The syringe adapter membrane is moveable from a first position within the housing of the syringe adapter to a second positon within the housing when the patient connector is positioned within the housing of the syringe adapter. The membrane of the patient connector is configured to engage the syringe adapter membrane. The cannula is configured to puncture the membrane of the patient connector when the patient connector is positioned within the housing of the syringe adapter.

### BRIEF DESCRIPTION OF THE DRAWINGS

The above-mentioned and other features and advantages of this disclosure, and the manner of attaining them, will become more apparent and the disclosure itself will be better understood by reference to the following descriptions of aspects of the disclosure taken in conjunction with the accompanying drawings, wherein:
FIG. 1 is a front view of a patient connector according to one aspect or embodiment of the present application.
FIG. 2 is a cross-sectional view of the patient connector of FIG. 1.
FIG. 3 is a cross-sectional view of the patient connector of FIG. 1, showing the patient connector being inserted into a syringe adapter.
FIG. 4 is a cross-sectional view of the patient connector of FIG. 1, showing the patient connector inserted into a syringe adapter.

Corresponding reference characters indicate corresponding parts throughout the several views. The exemplifications set out herein illustrate exemplary aspects of the disclosure, and such exemplifications are not to be construed as limiting the scope of the disclosure in any manner.

### DETAILED DESCRIPTION

The following description is provided to enable those skilled in the art to make and use the described aspects contemplated for carrying out the invention. Various modifications, variations, and alternatives, however, will remain readily apparent to those skilled in the art. Any and all such modifications, variations, and alternatives are intended to fall within the scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary aspects of the invention. Hence, specific dimensions and other physical characteristics related to the aspects disclosed herein are not to be considered as limiting.

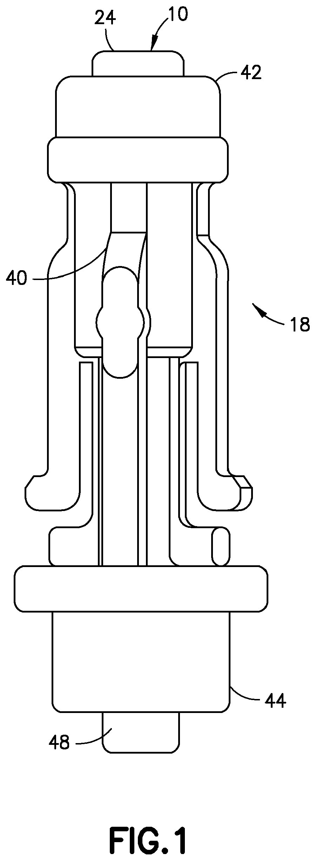

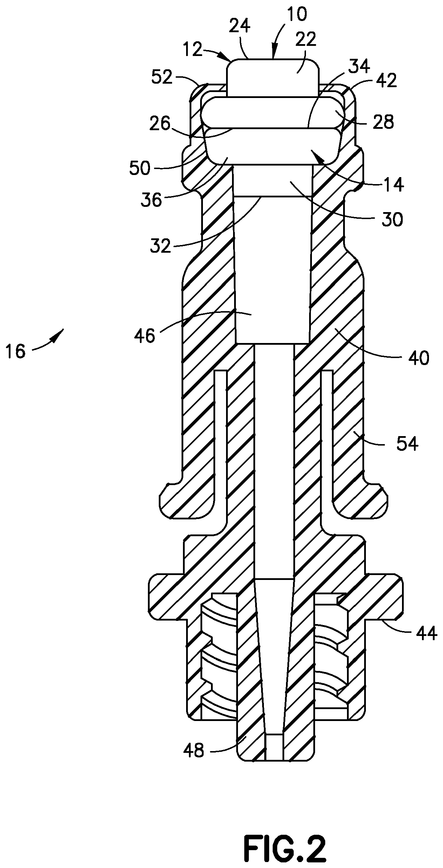

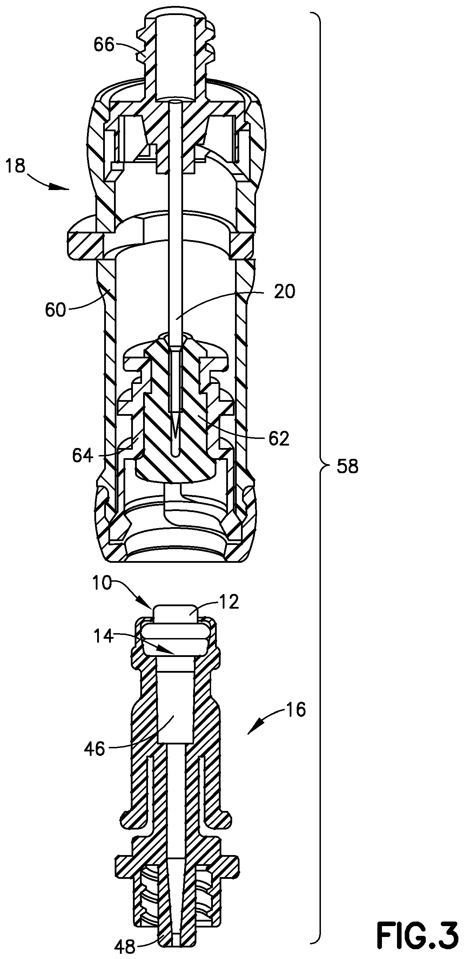

Referring to FIGS. 1-4, a membrane 10 for a closed system transfer device according to the invention includes a first portion 12 having a first material and second portion 14 having a second material. As shown in FIGS. 1 and 2, the membrane 10 is shown in connection with a patient connector 16, which is utilized to connect one component of a closed system transfer device or system to a patient intravenous line. For example, the patient connector 16 may be connected to a syringe adapter 18 to facilitate the transfer of fluid from one container, such as a syringe barrel, to another container or line, such as an intravenous line, IV bag, or other component. The membrane 10 may be utilized in any component of a closed system transfer device or system. In one aspect or embodiment, the membrane 10 is utilized in each component that mates with the syringe adapter 18, such as the patient connector 16, vial adapter, IV bag spike, etc. The syringe adapter 18 may be the same and operate in the same manner as the syringe adapter shown and described in United States Patent Application Publication No. 2015/0297454.

Referring to FIGS. 1 and 2, the first material of the first portion 12 of the membrane 10 has different material properties than the second material of the second portion 14. The first portion 12 is configured to prevent leakage through the first portion 12 when the first portion 12 is punctured by a cannula 20 for a first predetermined amount of time, and the second portion 14 is configured to prevent leakage through the second portion 14 when the second portion 14 is punctured by the cannula 20 for a second predetermined amount of time. The first predetermined amount of time is larger than the second predetermined amount of time. The first material is a thermoplastic elastomer, although other suitable materials may be utilized. In one aspect or embodiment, the first material may be DRYFLEX^{®} thermoplastic elastomer available from HEXPOL TPE. The second material is a polyisoprene, although other suitable materials may be utilized.

In one aspect or embodiment, the first predetermined amount of time is one hour or greater. In one aspect or embodiment, the second predetermined amount of time is 10 seconds or shorter. In certain configurations, the first predetermined amount of time could be on the order of 24 hours and intended to prevent "long-term" leakage, and the second predetermined about of time could be on the order of a few second to a minute for preventing "short-term" leakage. As noted above, the membrane 10 is utilized in connection with a closed system transfer device or system and, during use, the cannula 20 of the syringe adapter 18 may puncture the membrane 10 and quickly, such as a time period of 10 seconds or shorter, be withdrawn from the membrane. The membrane 10 may also be utilized in scenarios where the cannula 20 of the syringe adapter 18 punctures the membrane 10 and remains in the punctured position for an extended period of time, such as one hour or greater. The membrane 10 is configured to prevent leakage through the membrane 10, such as through an opening caused by the cannula 20 puncturing the membrane 10 or through an interface between the cannula 20 and the membrane 10. The first portion 12 of the membrane 10 is configured to optimize sealing performance for the scenario where the membrane 10 is punctured and remains punctured for the first predetermined amount of time. The second portion 14 of the membrane 10 is configured to optimize sealing performance for the scenario where the membrane 10 is punctured for the second predetermined amount of time. Accordingly, the membrane 10 with the first and second portions 12, 14 is configured to reduce leakage in both long-term and short-term puncturing scenarios.

In one aspect or embodiment, the first portion 12 and the second portion 14 are formed separately. The first portion 12 and the second portion 14 may also be formed integrally. The first portion 12 and the second portion 14 each include a body 22, 30 having a first side 24, 32 and a second side 26, 34 positioned opposite the first side 24, 32 with a flange 28, 36 extending from the body 22, 30. As shown in FIG. 2, the flange 28 of the first portion 12 abuts the flange 36 of the second portion 14. The first and second portions 12, 14 of the membrane 10 may be circular, although other suitable shapes and configurations may be utilized. The first portion 12 may be secured to the second portion 14 via an adhesive or any other suitable arrangement.

Referring again to FIGS. 1 and 2, the patient connector 16 includes a body 40 having a first end 42 and a second end 44, with the body 40 defining a passageway 46, a line connection 48 positioned at the second end 44 of the body 40, and the membrane 10 positioned at the first end 42 of the body 40. The line connection 48 may be a luer lock connection, although other suitable connections may be utilized. The membrane 10 is received by an opening 50 defined by the body 40 of the patient connector 16. The opening 50 of the patient connector 16 is wider than the passageway 46, with the body 22 of the first portion 12 of the membrane 10 extending from the first end 42 of the body 40 and the flange 28 of the first portion 12 positioned within the opening 50. The body 30 of the second portion 14 of the membrane 10 is positioned within the passageway 46 and the flange 36 of the second portion 14 is positioned within the opening 50. The body 40 of the patient connector 16 includes a securing extension 52 at the first end 42 of the body 40, with the securing extension 52 extending radially inward and configured to secure the membrane 10 to the body 40 of the patient connector 16. The patient connector 16 also includes a locking arrangement 54 configured to secure the patient connector 16 to the syringe adapter 18.

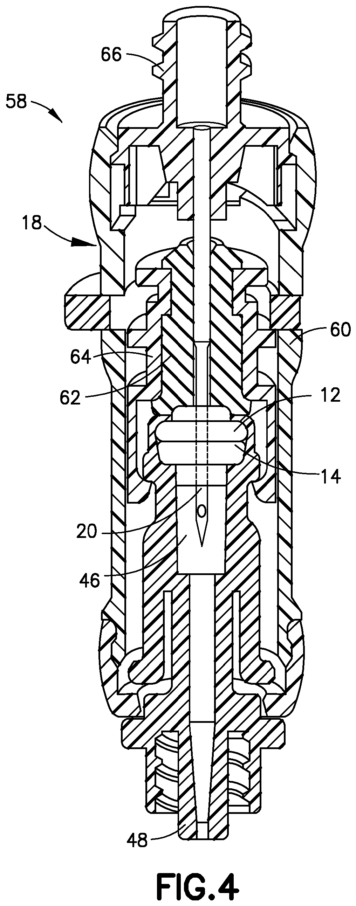

In a further aspect or embodiment, a system 58 for the closed transfer of fluid includes the patient connector 16 and the syringe adapter 18, although the system 58 may also include other components of a closed system transfer device or system. The syringe adapter 18 includes a housing 60 having a syringe adapter membrane 62 received within the housing 60 and the cannula 20. The syringe adapter membrane 62 is moveable from a first position within the housing 60 of the syringe adapter 18 to a second positon within the housing 60 when the patient connector 16 is positioned within the housing 60 of the syringe adapter 18, as shown in FIG. 4. The membrane 10 of the patient connector 16 is configured to engage the syringe adapter membrane 62. The cannula 20 is configured to puncture the membrane 10 of the patient connector 16 when the patient connector 16 is positioned within the housing 60 of the syringe adapter 18. The syringe adapter membrane 62 is received by a collet 64, although other suitable arrangements may be utilized. The syringe adapter 18 includes a luer connector 66 configured to be secured to a syringe barrel. The operation of the syringe adapter 18 is described in United States Patent Application Publication No. 2015/0297454.

While this disclosure has been described as having exemplary designs, the present disclosure can be further modified within the scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims. To the extent possible, one or more features of any aspect or embodiment discussed above can be combined with one or more features of any other aspect or embodiment.

## Claims

1. A membrane (10) for a closed system transfer device comprising:
a first portion (12) comprising a first body (22) and a first flange (28) extending from the first body (22), a first material wherein the first material comprises a thermoplastic elastomer; and
a second portion (14) comprising a second body (30) and a second flange (36) extending form the second body (30), a second material wherein the second material comprises a polyisoprene, the first material having different material properties than the second material, wherein the first portion (12) is configured to prevent leakage through the first portion (12) when the first portion (12) is punctured by a cannula for a first predetermined amount of time, and wherein the second portion (14) is configured to prevent leakage through the second portion (14) when the second portion (14) is punctured by the cannula for a second predetermined amount of time, the first predetermined amount of time is larger than the second predetermined amount of time,
**characterized in that**
the first body (22) has a width and the first flange (28) has a width, the width of the first flange (28) is wider than the width of the first body (22), the second body (30) has a width and the second flange (36) has a width, the width of the second flange (36) is wider than the width of the second body (30), and the first flange (28) abuts the second flange (36) such that the first flange (28) and the second flange (36) separate the first body (22) and the second body (30).

2. The membrane (10) of claim 1, wherein the first predetermined amount of time is one hour or greater.

3. The membrane (10) of any of claims 1-2, wherein the second predetermined amount of time is 10 seconds or shorter.

4. The membrane (10) of any of claims 1-3, wherein the first portion (12) and the second portion (14) are formed separately.

5. The membrane (10) of any of claims 1-4, wherein the first portion (12) and the second portion (14) are formed integrally.

6. The membrane (10) of any of claims 1-5, wherein the first body (22) has a first side and a second side positioned opposite the first side.

7. The membrane (10) of any of claims 1-6, wherein the second body (30) has a first side and a second side positioned opposite the first side.

8. A patient connector comprising:
a body having a first end and a second end, the body defining a passageway;
a line connection positioned at the second end of the body; and
a membrane (10) according to any of claims 1-7 positioned at the first end of the body.

9. The patient connector of claim 8, wherein the membrane (10) is received by an opening defined by the body.

10. The patient connector of claim 9, wherein the opening is wider than the passageway, the body of the first portion (12) extending from the first end of the body, the flange of the first portion positioned within the opening, the body of the second portion (14) positioned within the passageway, and the flange of the second portion (14) positioned within the opening.

11. The patient connector of claim 10, wherein the body of the patient connector comprises a securing extension at the first end of the body, the securing extension extending radially inward and configured to secure the membrane (10) to the body.

12. A system for the closed transfer of fluid comprising:
a patient connector according to any of claims 8-11; and
a syringe adapter comprising a housing having a syringe adapter membrane (62) received within the housing and a cannula, the syringe adapter membrane (62) moveable from a first position within the housing of the syringe adapter to a second positon within the housing when the patient connector is positioned within the housing of the syringe adapter,
wherein the membrane (10) of the patient connector is configured to engage the syringe adapter membrane (62), and wherein the cannula is configured to puncture the membrane (10) of the patient connector when the patient connector is positioned within the housing of the syringe adapter.

## Patentansprüche

1. Membran (10) für eine Transfervorrichtung mit geschlossenem System, welche aufweist:
einen ersten Teil (12), der einen ersten Körper (22) und einen ersten Flansch (28), der sich von dem ersten Körper (22) erstreckt, und ein erstes Material umfasst, wobei das erste Material ein thermoplastisches Elastomer umfasst; und
einen zweiten Teil (14), der einen zweiten Körper (30) und einen zweiten Flansch (36) umfasst, der sich von dem zweiten Körper (30) aus erstreckt, ein zweites Material, wobei das zweite Material ein Polyisopren umfasst, wobei das erste Material andere Materialeigenschaften als das zweite Material aufweist,
wobei der erste Teil (12) so ausgebildet ist, dass er eine Leckage durch den ersten Teil (12) hindurch verhindert, wenn der erste Teil (12) für eine erste vorbestimmte Zeitspanne von einer Kanüle durchstochen wird, und wobei der zweite Teil (14) so ausgebildet ist, dass er eine Leckage durch den zweiten Teil (14) verhindert, wenn der zweite Teil (14) durch die Kanüle für eine zweite vorbestimmte Zeitspanne durchstochen wird, wobei die erste vorbestimmte Zeitspanne größer ist als die zweite vorbestimmte Zeitspanne,
**dadurch gekennzeichnet,**
**dass** der erste Körper (22) eine Breite und der erste Flansch (28) eine Breite hat. Die Breite des ersten Flansches (28) breiter ist als die Breite des ersten Körpers (22), der zweite Körper (30) eine Breite und der zweite Flansch (36) eine Breite hat, die Breite des zweiten Flansches (36) breiter ist als die Breite des zweiten Körpers (30), und der erste Flansch (28) an dem zweiten Flansch (36) derart anliegt, dass der erste Flansch (28) und der zweite Flansch (36) den ersten Körper (22) und den zweiten Körper (30) trennen.

2. Membran (10) nach Anspruch 1, bei welcher die erste vorbestimmte Zeitspanne eine Stunde oder mehr beträgt.

3. Membran (10) nach einem der Ansprüche 1-2, bei welcher die zweite vorbestimmte Zeitspanne 10 Sekunden oder kürzer ist.

4. Membran (10) nach einem der Ansprüche 1-3, bei welcher der erste Abschnitt (12) und der zweite Abschnitt (14) separat ausgebildet sind.

5. Membran (10) nach einem der Ansprüche 1-4, bei welcher der erste Teil (12) und der zweite Teil (14) einstückig ausgebildet sind.

6. Membran (10) nach einem der Ansprüche 1-5, bei welcher der erste Körper (22) eine erste Seite und eine zweite Seite aufweist, die der ersten Seite gegenüberliegt.

7. Membran (10) nach einem der Ansprüche 1-6, bei welcher der zweite Körper (30) eine erste Seite und eine zweite Seite aufweist, die der ersten Seite gegenüberliegt.

8. Patientenkonnektor, welcher aufweist:
einen Körper mit einem ersten Ende und einem zweiten Ende, wobei der Körper einen Durchgang definiert;
eine Leitungsverbindung, die am zweiten Ende des Körpers angeordnet ist; und
eine Membran (10) nach einem der Ansprüche 1-7, die am ersten Ende des Körpers angeordnet ist.

9. Patientenkonnektor nach Anspruch 8, bei welchem die Membran (10) von einer durch den Körper definierten Öffnung aufgenommen ist.

10. Patientenkonnektor nach Anspruch 9, bei welchem die Öffnung breiter als der Durchgang ist, der Körper des ersten Teils (12) sich vom ersten Ende des Körpers erstreckt, der Flansch des ersten Teils innerhalb der Öffnung positioniert ist, der Körper des zweiten Teils (14) innerhalb des Durchgangs positioniert ist und der Flansch des zweiten Teils (14) innerhalb der Öffnung positioniert ist.

11. Patientenkonnektor nach Anspruch 10, bei welchem der Körper des Patientenkonnektors eine Befestigungsverlängerung am ersten Ende des Körpers umfasst, wobei sich die Befestigungsverlängerung radial nach innen erstreckt und so ausgebildet ist, dass sie die Membran (10) am Körper befestigt.

12. System für den geschlossenen Transfer von Flüssigkeiten, welches aufweist:
einen Patientenkonnektor nach einem der Ansprüche 8-11; und
einen Spritzenadapter, der ein Gehäuse mit einer Spritzenadaptermembran (62), die innerhalb des Gehäuses aufgenommen ist, und eine Kanüle umfasst, wobei die Spritzenadaptermembran (62) von einer ersten Position innerhalb des Gehäuses des Spritzenadapters zu einer zweiten Position innerhalb des Gehäuses bewegbar ist, wenn der Patientenkonnektor innerhalb des Gehäuses des Spritzenadapters positioniert ist,
wobei die Membran (10) des Patientenkonnektors so ausgebildet ist, dass sie in die Membran (62) des Spritzenadapters eingreift, und wobei die Kanüle so ausgebildet ist, dass sie die Membran (10) des Patientenkonnektors durchsticht, wenn der Patientenkonnektor innerhalb des Gehäuses des Spritzenadapters positioniert ist.

## Revendications

1. Membrane (10) pour un dispositif de transfert de système fermé comprenant :
une première partie (12) comprenant un premier corps (22) et une première bride (28) s'étendant à partir du premier corps (22), un premier matériau, le premier matériau comprenant un élastomère thermoplastique ; et
une deuxième partie (14) comprenant un deuxième corps (30) et une deuxième bride (36) s'étendant à partir du deuxième corps (30), un deuxième matériau, le deuxième matériau comprenant un polyisoprène, le premier matériau ayant des caractéristiques de matériau différentes de celles du deuxième matériau, la première partie (12) étant configurée pour empêcher une fuite à travers la première partie (12) lorsque la première partie (12) est ponctionnée par une canule pendant un premier laps de temps prédéterminé, et la deuxième partie (14) étant configurée pour empêcher une fuite à travers la deuxième partie (14) lorsque la deuxième partie (14) est ponctionnée par la canule pendant un deuxième laps de temps prédéterminé, le premier laps de temps prédéterminé étant plus long que le deuxième laps de temps prédéterminé,
**caractérisée en ce que**
le premier corps (22) a une largeur et la première bride (28) a une largeur, la largeur de la première bride (28) étant plus grande que la largeur du premier corps (22), le deuxième corps (30) a une largeur et la deuxième bride (36) a une largeur, la largeur de la deuxième bride (36) étant plus grande que la largeur du deuxième corps (30), et la première bride (28) jouxte la deuxième bride (36) de façon que la première bride (28) et la deuxième bride (36) séparent le premier corps (22) du deuxième corps (30).

2. Membrane (10) selon la revendication 1, le premier laps de temps prédéterminé étant une heure ou plus.

3. Membrane (10) selon l'une des revendications 1 à 2, le deuxième laps de temps prédéterminé étant 10 secondes ou moins.

4. Membrane (10) selon l'une des revendications 1 à 3, la première partie (12) et la deuxième partie (14) sont formées séparément.

5. Membrane (10) selon l'une des revendications 1 à 4, la première partie (12) et la deuxième partie (14) sont formées d'une seule pièce.

6. Membrane (10) selon l'une des revendications 1 à 5, le premier corps (22) ayant un premier côté et un deuxième côté positionné à l'opposé du premier côté.

7. Membrane (10) selon l'une des revendications 1 à 6, le deuxième corps (30) ayant un premier côté et un deuxième côté positionné à l'opposé du premier côté.

8. Connecteur de patient comprenant :
un corps ayant une première extrémité et une deuxième extrémité, le corps définissant un passage ;
une connexion de conduit positionnée à la deuxième extrémité du corps ; et
une membrane (10) selon l'une des revendications 1 à 7 positionnée à la première extrémité du corps.

9. Connecteur de patient selon la revendication 8, la membrane (10) étant reçue par une ouverture définie par le corps.

10. Connecteur de patient selon la revendication 9, l'ouverture étant plus large que le passage, le corps de la première partie (12) s'étendant à partir de la première extrémité du corps, la bride de la première partie étant disposée dans l'ouverture, le corps de la deuxième partie (14) étant disposé dans le passage et la bride de la deuxième partie (14) étant positionnée dans l'ouverture.

11. Connecteur de patient selon la revendication 10, le corps du connecteur de patient comprenant une extension de sécurité à la première extrémité du corps, l'extension de sécurité s'étendant radialement vers l'intérieur et étant configurée pour sécuriser la membrane (10) sur le corps.

12. Système pour le transfert fermé d'un fluide comprenant :
un connecteur de patient selon l'une des revendications 8 à 11 ; et
un adaptateur de seringue comprenant un boîtier ayant une membrane d'adaptation de seringue (62) reçue dans le boîtier et une canule, la membrane d'adaptation de seringue (62) pouvant être déplacée d'une première position dans le boîtier de l'adaptateur de seringue à une deuxième position dans le boîtier lorsque le connecteur de patient est positionné dans le boîtier de l'adaptateur de seringue,
la membrane (10) du connecteur de patient étant configurée pour entrer en contact avec la membrane d'adaptation de seringue (62), et la canule étant configurée pour perforer la membrane (10) du connecteur de patient lorsque le connecteur de patient est positionné dans le boîtier de l'adaptateur de seringue.
